Europäisches Patentamt

(19) European Patent Office

Office européen des brevets

(11) Numéro de publication : **0 260 181 B1**

(12) **FASCICULE DE BREVET EUROPEEN**

(51) Int. Cl.⁵ : **A61K 35/78**

(45) Date de publication du fascicule du brevet :
**05.06.91 Bulletin 91/23**

(21) Numéro de dépôt : **87401950.8**

(22) Date de dépôt : **01.09.87**

(54) Procédé de fabrication d'une poudre à grande efficacité cicatrisante, à partir de l'écorce de l'arbre dénommé "mimosa tenuiflora poir".

(30) Priorité : **09.09.86 MX 298**

(43) Date de publication de la demande :
**16.03.88 Bulletin 88/11**

(45) Mention de la délivrance du brevet :
**05.06.91 Bulletin 91/23**

(84) Etats contractants désignés :
**DE ES FR GB IT**

(56) Documents cités :
- -

(73) Titulaire : **Roque, Onel Léon Léon
Buenavista No. 46 Col. Lindavista
MEXICO D.F. 07300 (MX)**
Titulaire : **de Barrau, Jean
47, Faubourg Madeleine
F-45014 Orléans (FR)**
Titulaire : **Jacquemin-Sablon, Jean-Luc
Valcreuse
F-86270 La Roche Posay (FR)**

(72) Inventeur : **Roque, Onel Leon Leon
Buenavista No. 46 Col. Lindavista
Mexico, D.F. 07300 (MX)**

(74) Mandataire : **Descourtieux, Philippe et al
CABINET BEAU de LOMENIE 55 rue
d'Amsterdam
F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

On sait qu'un certain nombre de plantes possèdent des propriétés thérapeutiques. Il en est ainsi de l'écorce de l'arbre "Mimosa Tenuiflora poir" qui contient des substances dotées d'une efficacité cicatrisante.

La présente invention a pour objet un procédé de fabrication, à partir de l'écorce de l'arbre précité, d'une poudre susceptible d'être utilisée pour le traitement de certaines affections de la peau, notamment des brûlures.

Selon l'invention, on prélève tout d'abord des morceaux d'écorce du "Mimosa Tenuiflora poir" en choisissant des arbres âgés d'au moins 8 ans et de préférence âgés de 22 à 27 ans. Les dimensions de ces morceaux d'écorce seront adaptées au matériel dont on disposera pour effectuer les étapes suivantes du procédé, mais il est avantageux que les dimensions dans le sens des fibres du bois ne dépassent pas 20 cm et soient de préférence comprises entre 5 et 15 cm.

Le "Mimosa Tenuiflora poir" dont l'écorce présente les propriétés recherchées se trouve notamment au Mexique, dans la région d'Ismo Alto, à une altitude comprise entre 450 et 700 m.

Après séchage au soleil, l'écorce récoltée contient environ 15% d'eau qu'il convient d'éliminer, ce qui résultera, au moins indirectement, des étapes suivantes du procédé de fabrication. On procède en effet tout d'abord à une stérilisation de l'écorce récoltée. Dans ce but, on la chauffe, dans un récipient fermé, par exemple un récipient autoclave, à une température comprise entre 100 et 150°C, la pression ne devant pas cependant dépasser 5 bars. Pour conserver un temps raisonnable de stérilisation, la pression sera avantageusement maintenue au-dessus de 3 bars, la durée de cette phase du procédé étant alors comprise entre 1 et 2 heures. Il a été observé qu'une stérilisation effectuée à 125°C, sous une pression de 4 bars pendant 1,5 heure, donne des résultats tout à fait satisfaisants.

Après cette opération, le taux d'humidité résiduel de l'écorce peut encore atteindre 1%. Il est avantageux d'éliminer cette humidité résiduelle et, à cet effet, on procédera, si nécessaire, à un chauffage de l'écorce, à l'air libre, à une température comprise entre 120 et 130°C pendant au maximum 10 minutes, de préférence 6 à 7 minutes.

Après sa stérilisation, éventuellement suivie de l'élimination de l'humidité résiduelle, l'écorce est soumise à un grillage par chauffage intense, à l'air libre, une température comprise entre 130 et 140°C. Le temps de grillage est de l'ordre de 20 minutes, mais est d'autant plus court que la température est plus élevée. On a, par exemple, constaté que des résultats satisfaisants sont obtenus avec une température de 140°C maintenue pendant 17 minutes alors que 20 minutes sont nécessaires si la température est seulement 135°C.

Les produits actifs contenus dans l'écorce ayant été traités de la façon décrite, il convient de les séparer de la cellulose en éliminant cette dernière.

A cet effet, on soumet l'écorce à un broyage, par exemple au moyen d'un broyeur à marteaux, tournant à une vitesse comprise entre 1750 et 2000 tours/minute. Au moins un tamisage retient la majeure partie des fibres de cellulose et laisse passer le résidu granuleux contenant les produits actifs. Toutefois, il n'est, en général, pas possible d'éliminer totalement la cellulose, notamment les fibres courtes, par les tamis du broyeur, et un dernier étage d'élimination de la cellulose opérera avantageusement par attraction électrostatique.

Le résidu granuleux est alors amené à la granulométrie désirable par une réduction en poudre, par exemple au moyen d'un broyeur à cylindre ou tout autre appareil analogue. Il est cependant essentiel que la réduction en poudre soit suffisamment poussée pour que le résidu pulvérulent franchisse un tamis de 100 mailles par pouce, c'est-à-dire pratiquement 1600 orifices par centimètre carré. Ainsi, la poudre est constituée par un mélange de grains de diverses dimensions n'excédant pas 20 µm environ.

On a constaté que cette granulométrie, combinée aux produits actifs de la poudre, provoque une cicatrisation rapide de certaines plaies, avec régénération de la peau.

Cependant, il est possible d'augmenter l'efficacité de la poudre préparée selon le procédé qui vient d'être décrit et l'invention a également pour objet un procédé de fabrication d'une poudre d'écorce de "Mimosa Tenuiflora poir" permettant de réduire notablement le temps de cicatrisation.

A cet effet, on réalise un mélange de la poudre dont le procédé de fabrication a été décrit plus haut, avec un filtrat concentré de cette même poudre. La réduction du temps de cicatrisation dépend des proportions du mélange qui seront choisies en fonction des applications envisagées et/ou des prescriptions médicales.

Dans un mode d'exécution avantageux, le filtrat concentré est obtenu à partir d'une dissolution de poudre dans un solvant choisi parmi l'alcool, l'éther de pétrole et le cyclohexane, à raison d'environ 80% en poids de solvant et de 20% de poudre. Le traitement de la poudre par le solvant est poursuivi pendant 10 à 15 minutes à une température comprise entre 45 et 55°C.

Après filtration, le solvant est évaporé à l'air libre, à une température ne dépassant pas 90°C, de préférence voisine de 75°C. L'extrait sec ainsi recueilli est sous forme colloïdale et peut contenir jusqu'à 21% des produits actifs présents dans la poudre traitée.

## Revendications

1. Procédé de fabrication, à partir de l'écorce de l'arbre dénommé "Mimosa Tenuiflora poir", d'une poudre susceptible d'être utilisée pour le traitement de certaines affections de la peau, caractérisé en ce qu'il comporte les étapes suivantes :

a) on prélève des morceaux d'écorce sur des arbres âgés d'au moins 8 ans, de préférence âgés de 22 à 27 ans

b) on procède à une stérilisation de l'écorce, dans un récipient fermé, par chauffage à une température comprise entre 100 et 150°C, de préférence voisine de 125°C, la pression étant simultanément maintenue à une valeur comprise entre 3 et 5 bars, de préférence voisine de 4 bars, cette stérilisation étant poursuivie pendant un temps compris ente 1 et 2 heures, de préférence 1,5 heure.

c) après sa stérilisation, on soumet l'écorce à un grillage à l'air libre, par chauffage intense à une température comprise entre 135 et 140°C pendant un temps compris entre 20 et 17 minutes, d'autant plus court que la température est plus élevée

d) on procède enfin à une élimination mécanique de la cellulose contenue dans l'écorce et le résidu est réduit en poudre, la grosseur maximale de grains étant déterminée par le passage dans un tamis comportant au moins 100 mailles.

2. Procédé selon la revendication 1, caractérisé en ce que, entre la stérilisation et le grillage, on applique un chauffage supplémentaire, à l'air libre, à une température comprise entre 120 et 130°C pendant 6 à 7 minutes.

3. Procédé selon la revendication 1, caractérisé en ce que l'élimination de la cellulose est obtenue par un broyage de l'écorce, par exemple au moyen d'un broyeur à marteaux, suivi d'au moins un tamisage, puis d'une élimination complète par attraction électrostatique.

4. Procédé selon la revendication 3, caractérisé en ce que le résidu pulvérulent de l'écorce est à nouveau broyé pour fractionner les gros grains et obtenir la granulométrie désirée.

5. Procédé pour augmenter l'efficacité de la poudre obtenue par le procédé selon l'une quelconque des revendications 1 à 4, caractérisé en ce que l'on réalise un mélange de ladite poudre avec un filtrat concentré de cette poudre.

6. Procédé selon la revendication 5, caractérisé en ce que le filtrat concentré est obtenu par dissolution de la poudre dans un solvant choisi parmi l'alcool, l'éther de pétrole et le cyclohexane, à raison d'environ 80% en poids de solvant et de 20% de poudre, cette dissolution étant effectuée pendant 10 à 15 minutes à une température comprise entre 45 et 55°C, puis suivie d'une filtration et d'une évaporation à l'air libre du solvant contenu dans le filtrat.

7. Procédé selon la revendication 6, caractérisé en ce que l'évaporation du solvant s'effectue à la pression atmosphérique et sous une température au plus égale à 90°C, de préférence comprise entre 70 et 75°C.

## Ansprüche

1. Verfahren zur Herstellung eines Puders aus der Rinde des Baumes "Mimosa Tenuiflora poir", welches zur Behandlung von bestimmten Hauterkrankungen verwendet werden kann, dadurch gekennzeichnet, daß es die folgenden Schritte umfaßt :

a) man entnimmt Rindenstücke von mindestens acht, vorzugsweise 22 bis 27 Jahre alten Bäumen ;

b) man nimmt eine Sterilisierung der Rinde in einem geschlossenen Behälter durch Erhitzen auf eine Temperatur zwischen 100 und 150°C, vorzugsweise im Bereich von 125°C, vor, wobei der Druck gleichzeitig auf einen Wert zwischen 3 und 5 bar, vorzugsweise im Bereich von 4 bar, gehalten wird, welche Sterilisierung während eines Zeitraums von 1 bis 2 Stunden, vorzugsweise 1,5 Stunden, stattfindet ;

c) nach der Sterilisierung unterzieht man die Rinde einer Röstung an freier Luft durch starkes Erhitzen auf eine Temperatur zwischen 135 und 140°C während eines Zeitraums zwischen 20 und 17 min., u.zw. umso kürzer, je höher die Temperatur ist ;

d) schließlich entfernt man mechanisch in der Rinde enthaltene Zellulose und reduziert den Rückstand zu Puder, wobei die maximale Körnchengröße durch Durchlassen durch ein Sieb mit mindestens 100 Maschen bestimmt wird.

2. Verfahren nach Anspruch 1, dadurch gekennzeichnet, daß zwischen der Sterilisierung und der Röstung ein zusätzliches Aufheizen auf eine Temperatur zwischen 120 und 130°C während 6 bis 7 min an freier Luft vorgenommen wird.

3. Verfahren nach Anspruch 1, dadurch gekennzeichnet, daß die Entfernung der Zellulose durch Mahlen der Rinde, beispielsweise mittels einer Hammermühle bewirkt wird, gefolgt von mindestens einer Siebung und dann der kompletten Entfernung durch elektrostatische Anziehung.

4. Verfahren nach Anspruch 3, dadurch gekennzeichnet, daß der pulverige Rückstand der Rinde zum Zerkleinern der großen Körner und Erzielung der gewünschten Granulometrie nochmals gemahlen wird.

5. Verfahren zur Erhöhung der Wirksamkeit des mit dem Verfahren nach einem der Ansprüche 1 bis 4 erhaltenen Puders, dadurch gekennzeichnet, daß man eine Mischung des Puders mit einem konzentrierten Filtrat dieses Puders herstellt.

6. Verfahren nach Anspruch 5, dadurch gekennzeichnet, daß das konzentrierte Filtrat durch Auflösen des Puders in einem Lösungsmittel, ausgewählt aus Alkohol, Petrolether und Cyclohexan, in einer Menge von etwa 80 Gew.% Lösungsmittel und 20 Gew.% Puder erhalten wird, wobei die Auflösung während 10 bis 15 min bei einer Temperatur zwischen 45 und 55°C mit anschließender Filtration und Abdampfung des im Filtrat enthaltenen Lösungsmittels an freier Luft durchgeführt wird.

7. Verfahren nach Anspruch 6, dadurch gekennzeichnet, daß die Abdampfung des Lösungsmittels unter Atmosphärendruck und bei einer Temperatur von höchstens 90°C, vorzugsweise zwischen 70 und 75°C, erfolgt.

## Claims

1. Process for the production, from the bark of the tree called "Mimosa Tenuiflora poir", of a powder which can be used for treating certain skin disorders, characterized in that it comprises the following steps:

a) taking pieces of bark from trees aged at least 8 years, preferably aged between 22 and 27 years,

b) sterilizing the bark in a closed container, by heating to a temperature comprised between 100 and 150°C, preferably about 125°C, the pressure being simultaneously kept to a value comprised between 3 and 5 bars, preferably around 4 bars, said sterilization being continued for a period comprised between 1 and 2 hours, preferably 1.5 hours

c) subjecting the bark after its sterilization to a grilling in the open air by intensive heating to a temperature comprised between 135 and 140°C for a period comprised between 20 and 17 minutes, all the shorter as the temperature is higher

d) finally, proceeding to the mechanical elimination of the cellulose contained in the bark and reducing the residue to a powder, the maximum size of the particles being determined by passage through a 100-mesh sieve

2. Process according to claim 1, characterized in that between sterilization and grilling, an additional heating is applied, in the open air, to a temperature comprised between 120 and 130°C for 6 to 7 minutes.

3. Process according to claim 1, characterized in that the elimination of the cellulose is achieved by a grinding of the bark, with, for example, a hammer mill, followed by at least one sieving operation and finally a complete elimination by electrostatic attraction.

4. Process according to claim 3, characterized in that the powder residue from the bark is ground once more in order to split the large particles and to obtain the desired granulometry.

5. Process for increasing the efficiency of the powder obtained by the process according to any one of claims 1 to 4, characterized in that a mixture of said powder with a concentrated filtrate thereof is prepared.

6. Process according to claim 5, characterized in that the concentrated filtrate is obtained by dissolving the powder in a solvent selected from alcohol, petroleum ether and cyclohexane, in the proportion of about 80% by weight of solvent and 20% by weight of powder, this dissolution being carried out for 10 to 15 minutes at a temperature comprised between 45 and 55°C, and is followed by a filtration and evaporation in the open air of the solvent contained in the filtrate.

7. Process according to claim 6, characterized in that the evaporation of the solvent is carried out at the atmospheric pressure and at a temperature at the most equal to 90°C, preferably comprised between 70 and 75°C.